# EUROPEAN PATENT SPECIFICATION

(11) **EP 2 556 829 B1**
(45) Date of publication and mention of the grant of the patent: **19.08.2015**
(21) Application number: 11177165.5
(22) Date of filing: 10.08.2011
(51) Int. Cl.: A61K 31/35, A61P 11/00

(54) **Bicyclic labdane diterpenes for use in the treatment of TRPC6 associated diseases**
Bicyclische Labdan-Diterpene zur Verwendung bei der Behandlung von Erkrankungen im Zusammenhang mit TRPC6
Diterpènes de labdane bicycliques à utiliser dans le traitement des maladies associées au TRPC6

(43) Date of publication of application: 13.02.2013
(73) Proprietor: Universität Leipzig, 04109 Leipzig (DE)
(72) Inventor: Schaefer, Michael, 04105 Leipzig (DE); Urban, Nicole, 04600 Altenburg (DE); Kübler, Wolfgang, Toronto M1N 2R3 (CA)
(74) Representative: Kailuweit & Uhlemann Patentanwälte Partnerschaft mbB

(56) References cited:
- WO-A1-96/24684
- WO-A2-2006/076735
- DE-A1- 19 853 476
- US-A1- 2006 257 500
- US-A1- 2010 137 194
- PFERSCHY-WENZIG EVA M ET AL: "In Vitro Anti-inflammatory Activity of Larch (Larix decidua L.) Sawdust", JOURNAL OF AGRICULTURAL AND FOOD CHEMISTRY, vol. 56, no. 24, December 2008 (2008-12), pages 11688-11693, XP002664255, ISSN: 0021-8561

## Description

The present invention relates to bicyclic labdane diterpenes for use in the treatment of TRPC6 associated diseases, in particular pulmonary hypertension, chronic pulmonary obstruction, obstructive airway diseases with an allergic origin, acute respiratory distress syndromes or renal diseases.

Transient receptor potential cation channel (TRPC) proteins are Ca²⁺-permeable, plasma membrane-resident cation channels that are activated in a cell surface receptor- and phospholipase C-dependent mechanism. Besides their physiological activation mechanism, TRPC channels can be activated or positively modulated by several other queues, including membrane-permeable diacylglycerols, natural compounds (hypericin), lanthanides, acidic conditions, or physical stretch. The genetically closely related subgroup consisting of TRPC3, TRPC6 and TRPC7 shares the unique property of being directly activated by diacylglycerol (DAG). TRPC6, as well as its closest relatives TRPC3 and TRPC7 are second messenger-gated, poorly selective cation channels. The expression of TRPC channels is widespread, but differs between the isoforms.

The TRPC6 isoform of canonical TRPC channels has recently been identified as a key player in pulmonary diseases such as hypoxia-induced pulmonary vasoconstriction (HPV) and renal diseases like focal segmental glomerulosclerosis (FSGS; US 2008/0038365 A1). In addition, dysregulated TRPC6 has been suggested to account for idiopathic pulmonary arterial hypertension and a critical role of TRPC6 has been proposed in chronic obstructive and allergic airway diseases. The development of pulmonary edema during hypoxia or acute lung injury has been described to be partly caused by TRPC6-dependent processes. Hence, blocking of TRPC6 channels is applicable in the treatment of a variety of diseases.

Various treatments for pulmonary hypertension are suggested to date. Endothelin receptor antagonists, nitric oxide (NO) gas or NO-releasing compounds, and phosphodiesterase inhibitors are available to counteract pulmonary vasoconstriction. Endothelin receptor antagonists can only block effects of one class of G-protein-coupled receptors, while responses to other vasoconstrictors remain unaffected. Alternative strategies converge on the level of cyclic guanosine 3',5'-monophosphyte (cGMP) either by increasing its formation by NO-sensitive soluble guanylyl cyclases or by inhibiting its degradation by blocking cGMP-specific phosphodiesterases of the PDE5 family.

Further, a positive effect of oridonin, a tricyclic diterpene derived from the Chinese herb *Rabdosia rubesecens*, is described for the treatment of pulmonary hypertension (Wang 2009). It is described in the art that oridonin can lower pulmonary artery pressure effectively, and inhibit pulmonary artery structural remodeling by inducing smooth cell apoptosis via a mitochondria-dependent pathway. Inhibiting pulmonary TRPC channels provides another class of pharmacological target molecules that is given by a second messenger-gated cation channel, and thereby integrates the inputs of various vasoconstrictor responses.

However, the distinct TRPC proteins share a closely related structure. Within the seven mammalian TRPC channels, subfamilies of more closely related isoforms are discernible. The TRPC1/TRPC4/TRPC5 subfamily and the TRPC3/TRPC6/TRPC7 subfamily share about 40-65% of amino acid identity within their respective members, but only 21-28% of amino acid identity across the subfamilies. Similarly, the potential of forming heteromeric channel complexes is clearly more pronounced within the phylogenetically and structurally more closely related members of a subfamily. Due to the close relation between the TRPC proteins, selective inhibitors of distinct members of TRPC have to be provided for therapeutic applications that aim on targeting members of the TRPC family.

US 2008/0038365 A1 discloses synthetic TRPC channel inhibitors, like 2-aminodiphenylborate (2-APB), SKF-96365 or gadolinium cations, for treatment of pulmonary vasoconstriction and focal and segmental glomerulosclerosis.

WO 2009/05972 A2 discloses norgestimate as inhibitor of the TRPC3/TRPC6/TRPC7 subfamily and its therapeutic application in treatment of renal and pulmonary diseases. Selectivity for TRPC6 compared to TRPC3 or TRPC7 is not achieved.

WO 2006/7074802 A1 discloses selective inhibition of TRPC6 with inactive or dominant negative subunits of TRPC6. However, it is known in the art that subunits of the proteins of the TRPC3/TRPC6/TRPC7 subfamily assemble by forming heteromeric complexes. A treatment with inactive or dominant negative subunits of TRPC6 may thereby also inhibit TRPC3 or TRPC7.

Therefore, there is a need for providing selective blockers that modulate the activity of distinct isoforms or subgroups of TRPC isoforms for therapeutic applications.

It is the object of the invention to provide selective blockers of TRPC6 that are therapeutically applicable for treatment of TPRC6-associated diseases, like pulmonary hypertension.

The object is solved by the use of a bicyclic labdane diterpene for blocking calcium transport via transient receptor potential cation channel 6 (TRPC6).

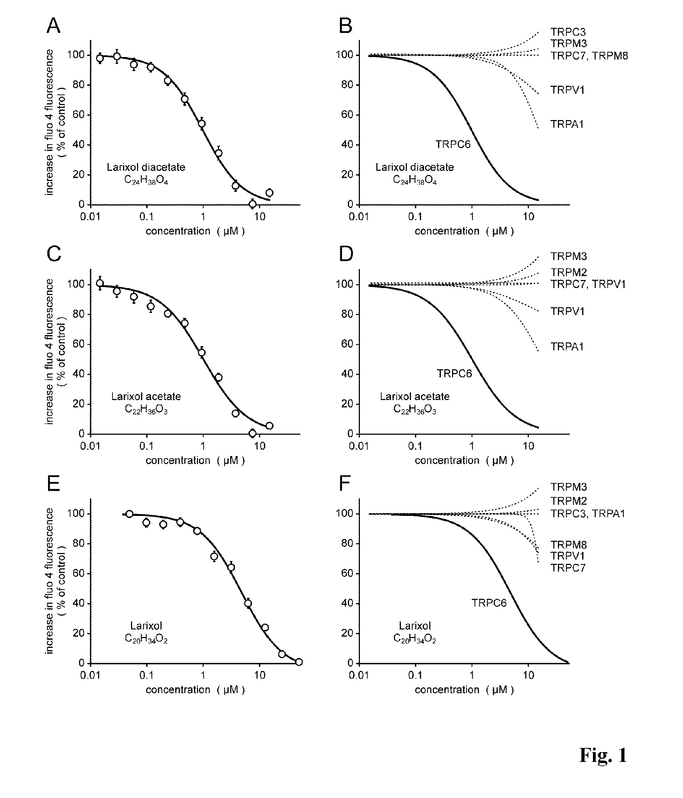

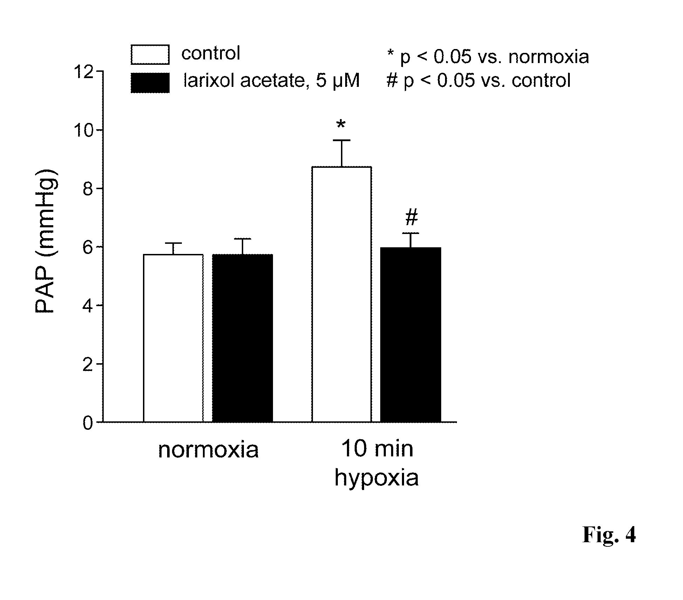

The inventors have found that bicyclic labdane diterpenes, especially labdane diterpenes structurally related to larixol, act as selective inhibitors for TRPC6 (Fig. 1). Bicyclic labdane diterpenes selectively bind to TRPC6 and show a significantly decreased binding to its structurally related subfamily members TRPC3 and TRPC7. It could be demonstrated in an induced mouse model that bicyclic labdane diterpenes are potent inhibitors of hypoxia-induced pulmonary vasoconstriction (HPV) (Fig. 4). Being a selective inhibitor of TRPC6, bicyclic labdane diterpenes are applicable for therapeutic treatment of diseases associated with activation of TRPC6.

Therefore, another aspect of the invention is a bicyclic labdane diterpene for use in the treatment of a disease associated with activation of TRPC6, preferably a pulmonary or a renal disease. Specifically, pulmonary and renal diseases that involve cell types and tissues that express TRPC6 are preferentially targeted with TRPC6 blockers. Particular pulmonary diseases that are treated include idiopathic or secondary pulmonary hypertension, chronic obstructive pulmonary disease (COPD), allergic airway disease, pulmonary vasoconstriction after lung transplantation, or acute respiratory distress syndrome (ARDS). Here, major cell types that, via TRPC6 activation or overexpression, contribute to the pathophysiological processes are vascular smooth muscle cells in large and small pulmonary vessels, smooth muscle cells of the airways, and immune cells that express TRPC6 and enhance inflammatory responses in pathological settings.

Particular renal diseases treated are focal segmental glomerulosclerosis, diabetic nephropathy, minimal change glomerulonephritis or preeclamptic nephropathy, particularly preferred is focal segmental glomerulosclerosis. In the kidney, dysregulated activity or expression of TRPC6 in podocytes causes podocyte malfunction, resulting in the development of focal segmental glomerulosclerosis.

Labdane diterpenes are natural diterpenes that share a bicyclic core structure according to the following formula (5). Based on this core structure, bi-, tri-, tetra- or multicyclic diterpene structures are formed.

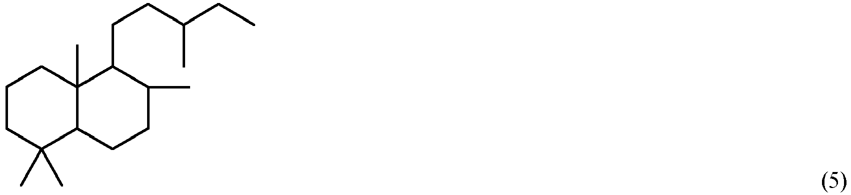

According to the invention labdane diterpenes with a bicyclic structure are used. Preferably, these bicyclic labdane diterpenes are isolated from conifers.

Therapeutic use of a distinct group of labdane diterpenes has been described in DE 198 53 476 A1 in agents lowering prolactin. These agents are applicable in treatment of premenstrual syndrome. However, therapeutic use of larixol-related bicyclic diterpenes is not described.

Hence, another aspect of the invention is a bicyclic labdane diterpene according to formula (1) for use as a medicament, preferably for use in the treatment of a pulmonary disease or renal disease (particularly preferred as defined above).

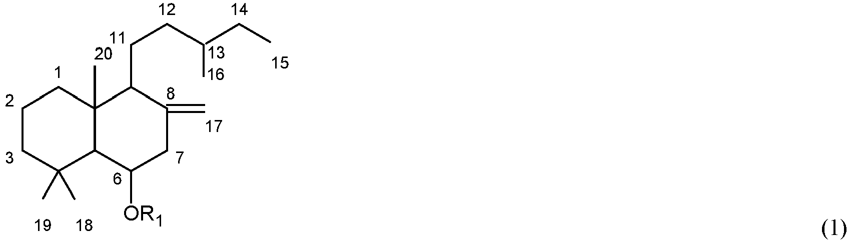

According to the invention, in formula (1) R₁ is selected from hydrogen and C₁ to C₄ acyl, preferably hydrogen or C₁ to C₃ acyl, more preferably hydrogen or COCH₃. According to the invention the bicyclic labdane diterpene according to formula (1) optionally comprises at least one double bond between the carbon atoms at positions 1 and 2, 2 and 3, 5 and 6, 6 and 7 and/or 14 and 15, preferably at least between the carbon atoms at positions 14 and 15. According to the invention the carbon atoms at positions 1, 2, 3, 7, 11, 12, 13, 14, 15, 16, 17, 18, 19 or 20, preferably 1, 2, 3, 7, 13, 17, 18, 19 or 20, particularly preferred 1, 2, 3, 7 or 13, are connected to hydrogen atoms or comprise at least one substitution. Preferably maximal 5, more preferably maximal 3, of the aforementioned carbon atoms comprise a substitution. Preferred substitutions at positions 1, 2, 3, 7, 11, 12, 13, 14, 15, 16, 17, 18, 19 or 20 are selected from a halogen or -OR₂ with R₂ being hydrogen or C₁ to C₄ acyl, preferably hydrogen or C₁ to C₃ acyl, more preferably hydrogen or COCH₃. A preferred substitution at positions 1, 2, 3 or 7 is a keto group.

The bicyclic ring structure in formula (1) preferably contains at least one double carbon bond within the ring structure that is preferably located between at positions 1 and 2, 2 and 3, 5 and 6, 6 and 7.

Larixol related bicyclic labdane diterpenes are characterized by the double bond between carbon atoms at positions 8 and 17 in formula (1) and the oxygen-containing substitution OR₁ at the carbon atom at position 6. Preferably, the bicyclic labdane diterpene for use as a medicament comprises a double bond between the carbon atoms at positions 14 and 15. Preferably, the bicyclic labdane diterpene for use as a medicament comprises a functional group with the structure -OR₂ at the carbon atom at position 13, wherein with R₂ is hydrogen or C₁ to C₄ acyl, preferably hydrogen or C₁ to C₃ acyl, more preferably hydrogen or COCH₃.

Particularly preferred bicyclic labdane diterpenes are characterized by the following formula (2):

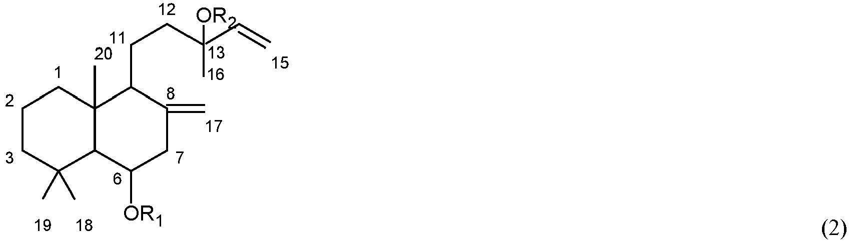

In formula (2) R₁ and R₂ are independent from each other selected from hydrogen and C₁ to C₄ acyl, preferably hydrogen or C₁ to C₃ acyl, more preferably hydrogen or COCH₃. Preferably the rests R₁ and R₂ are identical. In the particularly preferred bicyclic labdane diterpenes according to formula (2) the optional double bonds, substitutions and replacements as described for formula (1) are comprised. Preferably, bicyclic labdane diterpenes according to formula (2) optionally comprise at least one double bond between the carbon atoms at positions 1 and 2, 2 and 3, 5 and 6 and/or 6 and 7, optionally the carbon atoms at positions 1, 2, 3, 7, 11, 12, 14, 15, 16, 17, 18, 19 or 20, preferably 1, 2, 3, 7, 17, 18, 19 or 20, particularly preferred 1, 2, 3 or 7, are connected to hydrogen atoms or comprise at least one substitution. Preferred substitutions at positions 1, 2, 3, 7, 11, 12, 14, 15, 16, 17, 18, 19 or 20 selected from a halogen or -OR₂ with R₂ being hydrogen or C₁ to C₄ acyl, preferably hydrogen or C₁ to C₃ acyl, more preferably hydrogen or COCH₃. A preferred substitution at positions 1, 2, 3 or 7 is a keto group. Preferably, maximal 3 of the aforementioned carbon atoms of formula (2) comprise a substitution other than H.

Particularly preferred bicyclic labdane diterpenes are larixol and larixol acetates that are characterized respectively by formulas (3) and (4a, b and c):

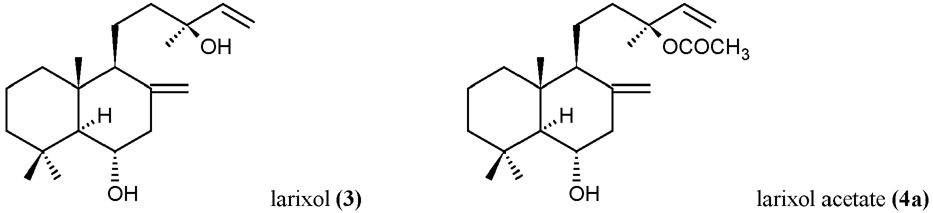

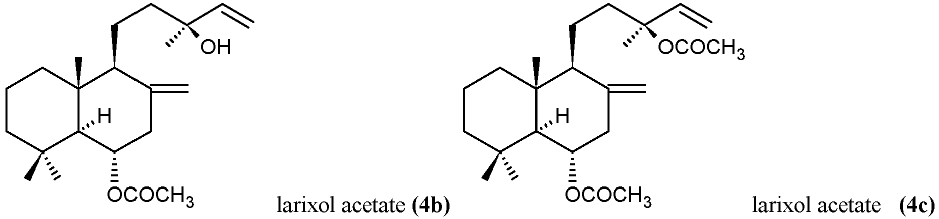

Another aspect of the invention is a method of treating a disease comprising the step of administering a therapeutically effective amount of a bicyclic labdane diterpene, preferably a larixol related bicyclic labdane diterpene as defined and preferred above, to a patient. Preferred diseases to be treated with a method according to the invention are diseases associated with TRPC6 activation, particularly preferred pulmonary or renal diseases. In a preferred method according to the invention, the bicyclic labdane diterpene is administered orally, by means of an inhalator or parenterally, preferably depending on the disease to be treated.

A further aspect of the invention is a pharmaceutical composition comprising a bicyclic labdane diterpene, preferably a larixol related bicyclic labdane diterpene as defined above, in combination with a pharmaceutically acceptable carrier.

The pharmaceutical compositions according to the invention include various dosage forms and are preferred for oral, inhalational or parenteral, particularly preferably suitable for intravenous administration. Preferably, the parenteral pharmaceutical composition is in a form which is suitable for injection. Particularly preferred pharmaceutical compositions are solutions, emulsions or suspensions of the bicyclic labdane diterpene in a pharmaceutically acceptable carrier. Pharmaceutically acceptable carriers are preferably sterile liquids, especially water, buffered water, 0.4% saline, 0.3% glycine and the like. The pharmaceutical compositions may be sterilized by conventional, well known techniques. The compositions preferably contain pharmaceutically acceptable auxiliary substances, such as those that are required to assure physiological conditions and/or that increase the stability of the contained bicylic diterpene. Preferred auxiliary substances are agents for adjusting the pH and buffering agents, preferably selected from sodium acetate, sodium chloride, potassium chloride, calcium chloride and sodium lactate.

The use of a bicyclic labdane diterpene for blocking calcium transport via TRPC6 may occur *in vivo*, e.g. by treating a patient suffering from a TRPC6 activation-associated disease, or *ex vivo.* In a preferred *ex vivo* use, the bicyclic labdane diterpene is comprised as active ingredient in a preservation solution that is used for storage or perfusion of isolated tissue. The term tissue as used herein includes isolated tissue as well as isolated complete organs. Hence, another aspect of the invention is the use of an aqueous solution (preservation solution), preferably a physiological solution, comprising a bicyclic labdane diterpene, preferably a larixol related bicyclic labdane diterpene as defined and preferred above, for storage and/or perfusion of tissue. In a preferred embodiment, an explanted tissue is stored and/or perfused with the preservation solution to prevent pulmonary vasoconstriction upon reperfusion. Before reimplantation, the preservation solution comprising the bicyclic labdane diterpene is preferably removed and replaced by a physiological solution being free of bicyclic labdane diterpene.

The invention provides a new class of TRPC channel inhibitors that selectively block calcium influx via TRPC6 and therefore are applicable in therapeutic treatment of patients with TRPC6-associated diseases. Bicyclic labdane diterpenes selectively block TRPC6 and do not inhibit calcium influx via the structurally closely related subfamily members TRPC3 and TRPC7. The bicyclic labdane diterpenes are natural compounds. No cytotoxic effects were assessed in *in vitro* and *in vivo* experiments.

The invention is further illustrated by the following figures and examples without being limited to these.
- Fig. 1: Selectivity of larixol and larixol acetate to block different isoforms of transient receptor potential cation channels (TRPC). Comparison of binding to human TRPC6, TRPC3, TRPA1 and murine TRPC6 shows selective binding to TRPC6. Other transient receptor potential cation channels such as TRPV1, TRPM8, TRPM2 and TRPM3 are not affected by larixol and larixol acetate.

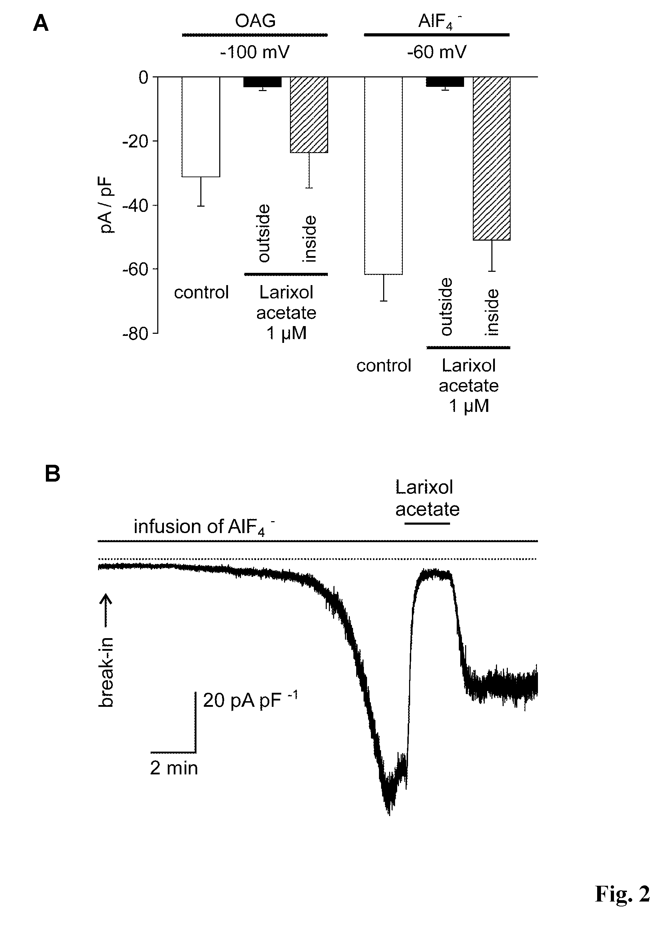

- Fig. 2: Larixol acetate acts from the outside of the cells. A: Electrophysiological whole-cell recordings were performed to determine the TRPC6-mediated current upon direct stimulation with 50 µM OAG or by activating heterotrimeric G-proteins with intracellularly applied AlF₄⁻ (50 µM), to trigger phospholipase C activation, resulting in the formation of endogenous diacylglycerols. To expose the cytosolic side of the channel protein to larixol acetate, larixol acetate was added to the intracellular solution (hatched bars and filled bars). Control solutions without larixol acetate (open bars) were measured in parallel. Intracellular exposure to larixol acetate (hatched bars) was not sufficient to suppress the OAG- or AlF₄⁻-induced TRPC6 activation, while larixol acetate applied to the bath solution ("outside"; filled bars) effectively blocked the channel activity. B: example of an original recording demonstrating the block of TRPC6 activity by larixol acetate when given via the bath solution.

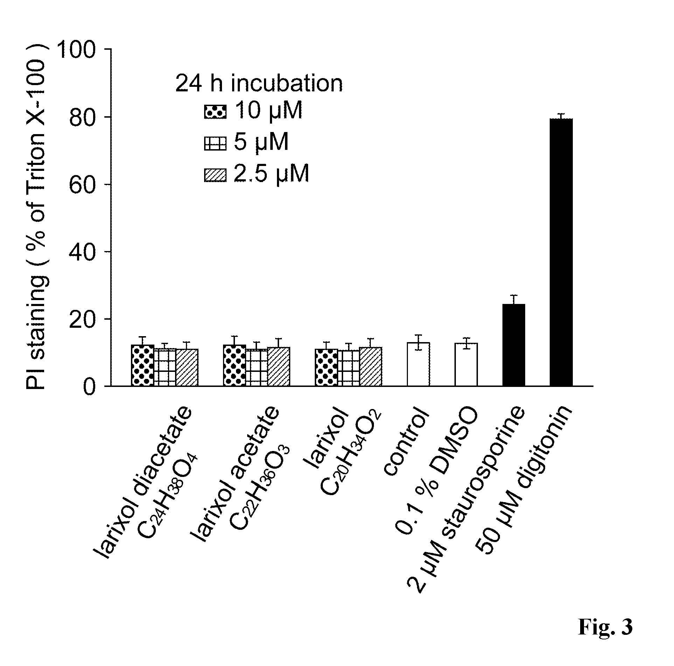

- Fig. 3: Larixol and larixol acetate to not show a cytotoxic effect on HEK293 cells. The cellular integrity was determined by exclusion of the DNA-binding fluorescent dye propidium iodide (PI).
- Fig. 4: Inhibition of hypoxia-induced pulmonary vasoconstriction (HPV) by larixol acetate. In isolated perfused mouse lungs, ventilation with hypoxic gas results in an increase in the pulmonary arterial pressure (PAP), which is completely abrogated when 5 µM larixol acetate was added to the perfusion solution.

### Example 1: Creation of TRPC-expressing HEK293 cells

HEK293 (ATCC CRL-1573) cells were grown in Earle's Minimum Essential Medium (MEM) supplemented with 10 Vol.-% fetal calf serum, 2 mM (mmol/l) L-glutamine, 100 units/ml penicillin and 0.1 mg/ml streptomycin. Cells were seeded in 35-mm culture dishes, grown for 24 h, and transfected at 80% confluence with 2 µg of plasmid DNA encoding a C-terminally YFP-tagged human TRPC6 and 4 µl of Fugene HD reagent (Roche) in 100 µl serum-free medium. To select stably transfected HEKhTRPC6-YFP clones, 1 mg/ml geneticin (G418) was added to the medium. Single clones were obtained by the limiting dilution method, and clones that exhibited a homogenous fluorescence were picked manually with optical control using an inverted epifluorescence microscope. To verify functional expression, different clones were isolated and tested by single-cell [Ca²⁺]ᵢ imaging experiments. The same procedure was also applied to create cell lines that express other TRP channel constructs. The following cell lines were created:

| **TRP channel construct** | **HEK293 cell line** |
|---|---|
| human TRPC6 | hTRPC6 |
| human TRPC3 | hTRPC3 |
| murine TRPC7 | mTRPC7 |
| human TRPA1 | hTRPA1 |
| human TRPM2 | hTRPM2 |
| rat TRPV1 | rTRPV1 |
| human TRPM8 | hTRPM8 |
| human TRPM3 | hTRPM3 |

All cells were grown at 37° C in a humidified atmosphere containing 5% CO₂.

### Example 2: Assessment of inhibitive action of larixol and larixol acetate on TRP channel constructs

To assess the effect of bicyclic labdane diterpenes on calcium transport via TRP channels, larixol and larixol acetate was added to cell lines from example 1. Blocking of the respective TRP channel was determined by determining increases in [Ca²⁺]ᵢ in suspensions. To activate the TRP channels, an activator of the respective channel was added, and changes in fluorescence intensity of intracellularly loaded fluo-4 was determined with a multiwell plate reader device (Polarstar Omega, BMG Labtech). For HEKrTRPV1 cells, assessment of activation was performed by monitoring the intracellular acidification, which results in fluorescence loss of co-expressed YFP.

The following experiments were performed in HEPES-buffered saline (HBS) containing 132 mM NaCl, 6 mM KCl, 1 mM MgCl₂, 1 mM CaCl₂, 5.5 mM glucose, and 10 mM HEPES adjusted to pH 7.4 with NaOH:
Pigmented clear-bottom 384-well screening plates (Corning, USA) were prefilled with HBS (26 µl/well), and serially diluted solutions of larixol and larixol acetate (4 µl/well to achieve final concentrations of 0.01 to 20 µM) were added. TRP channel expressing HEK293 cells from example 1 were grown to confluence, detached with 0.25% trypsin, and loaded for 30 min at 37°C in culture medium supplemented with 4 µM fluo-4/AM (Invitrogen). Cells were washed (100 x g; 3 min), resuspended in HBS, containing 0.1% bovine serum albumin (BSA), and the cell number was adjusted to 1000 cells/µl. Cell suspensions (10 µl/well) were dispensed into the prepared screening plates to obtain the final concentration of test compounds.

Fluorescence was detected at 485 nm excitation and 520 nm emission wavelengths in a multiwell plate reader (Polarstar Omega, BMG Labtech, Germany) applying a repeated on-the-fly plate scanning mode. Plates were scanned in two groups with 192 wells each. After 10 baseline cycles, OAG was injected with a final concentration of 50 µM. In all experiments, the fluorescence of tagged hTRPC6-YFP was negligible (< 1%) compared to fluorescence signals arising from the intracellularly loaded [Ca²⁺]ᵢ indicator dye. To generate concentration-response curves of primary hits or of reordered compounds, serial dilutions of compounds were tested in duplicates, each. TRPC6 was activated either with OAG or with a redundant mix of GPCR agonists, containing carbachol (1 mM), adenosine-5'-triphosphate (300 µM) and thrombin (0.5 U/ml). The combined stimulation with three agonists was applied to avoid effects of a possible receptor antagonism. To dissociate [Ca²⁺]ᵢ signals originating from store depletion and those of the TRPC6-dependent influx pathway, cells were preincubated with thapsigargin (2 µM for 5 min) prior to agonist injection. When GPCR agonists were applied, BSA was omitted from the HBS buffer. In specificity tests, concentration-response curves of compounds were determined in cell lines overexpressing other TRP channels. The respective activators were 50 µM OAG for TRPC3 and TRPC7, 1 mM H₂O₂ for TRPM2, 100 µM AITC for TRPA1, and 2 µM capsaicin for TRPV1.

The results of the study are shown in Fig. 1. Larixol and larixol acetate selectively inhibit calcium influx via TRPC6 whereas other TRP channels are not, or only poorly, inhibited.

### Example 3: Electrophysiological characterization of the mechanism of TRPC6 inhibition by larixol acetate

TRPC6-expressing cells were seeded on glass coverslips, mounted onto the stage of an inverted microscope, and contacted with a borosilicate glass pipet filled with an intracellular solution. After reaching a gigaohm seal, the whole-cell configuration was obtained by gently applying a negative pressure at the pipet. Whole-cell currents were measured in the voltage clamped mode with a holding potential of -60 mV, and normalized for the cell size by dividing current amplitudes by the cellular capacitance (pA/pF). TRPC6 was activated either by adding OAG to the bath solution or by including 50 µM AlF₄⁻ in the pipet solution. A typical example of TRPC6 activation is shown in the Fig. 2B.

In some experiments, the pipet solution was additionally supplemented with 1 µM larixol acetate to apply the modulator intracellularly (Fig. 2A, "inside"). An efficient block by 1 µM larixol acetate, however, was only observed when the blocker was applied via the bath solution (Fig. 2A "outside", Fig. 2B).

These experiments demonstrate that larixol acetate binds to the extracellularly exposed domain of TRPC6 and that intracellular signaling pathways are not required to exert TRPC6 blockade with larixol acetate.

### Example 4: Cytotoxicity of larixol and larixol acetate on HEK293 cells

To assess possible cytotoxic effects of the larixol and larixol acetate, a propidium iodide exclusion assay was performed. Cells were grown in 24 well plates for 18 h and then incubated with the indicated compounds for another 24 h (Fig. 3). Propidium iodide (2 µg/ml, Invitrogen) was added, and the fluorescence (excitation at 544 nm and detection at 620 nm) was followed. To obtain a maximum reference value, 0.2% Triton X-100 was added to some samples prior to propidium iodide injection. The following controls were tested:
- negative controls: HBS buffer ("control"), or 0,1 % DMSO in HBS buffer, added to the growth medium in the same volume as for 10 µM larixol ("0,1 % DMSO").
- positive controls: 2 µm staurosporine, 50 µm digitonin.

At concentrations up to 10 µM, no cytotoxic effect of larixol and larixol acetate on cells was discernible.

### Example 5: Inhibition of hypoxia-induced pulmonary vasoconstriction (HPV) by larixol acetate

To assess whether the TRPC6 blocking action of larixol acetate is potent to prevent HPV, larixol acetate was supplied in an induced mouse model of HPV.

Anesthetized, male adult C57BL/6 mice were subjected to tracheostomy, followed by volume-controlled normoxic ventilation. Midsternal thoracotomy was performed, and two cannulas (1 mm inner diameter) were inserted into the pulmonary artery and left atrium, respectively. Lungs were then perfused at a constant flow at 37°C with a Hanks' Balanced Salt Solution (Sigma-Aldrich) containing 5% bovine serum albumin (Sigma-Aldrich) and 5% dextran (Sigma-Aldrich) at pH of 7.4. Indomethacin (30 µM) and *N*^{G}-nitro-L-arginine methylester (1 mM) were added to the perfusate to inhibit endogenous prostaglandin and nitric oxide synthesis. Switching from a normoxic gas mixture of 21% O₂, 5% CO₂, in N₂ to a hypoxic hypoxic gas mixture containing 1% O₂, 5% CO₂, balance N₂, resulted in an increase in the pulmonary artery pressure (PAP) by about 40%. This well-known response, also referred to "von Euler-Lilljestrand mechanism", was completely abrogated when 5 µM of larixyl acetate were added to the perfusion solution (Fig. 4).

### Cited non patent literature

- Wang 2009: Wang LX, Sun Y, Chen C, Huang XY, Lin Q, Qian GQ, Dong W, Chen YF. Chin Med J (Engl). 2009 Jun 20;122(12):1380-7.

## Claims

1. Bicyclic labdane diterpene for use in the treatment of a pulmonary disease or renal disease by blocking calcium transport via the transient receptor potential cation channel 6.

2. Bicyclic labdane diterpene for use according to claim 1, wherein the pulmonary disease is idiopathic or secondary pulmonary hypertension, chronic obstructive pulmonary disease, an allergic airway disease, pulmonary vasoconstriction or an acute respiratory distress syndrome or the renal disease is a focal segmental glomerulosclerosis.

3. Use of bicyclic labdane diterpene for blocking calcium transport via the transient receptor potential cation channel 6 *ex vivo.*

4. Use according to claim 3 wherein the bicyclic labdane diterpene is comprised in an aqueous solution that is used as preservation solution for storage and/or perfusion of tissue.

5. Bicyclic labdane diterpene for use according to claim 1 or 2 or use according to claim 3 or 4, wherein the bicyclic labdane diterpene exhibits a structure according to formula (1) 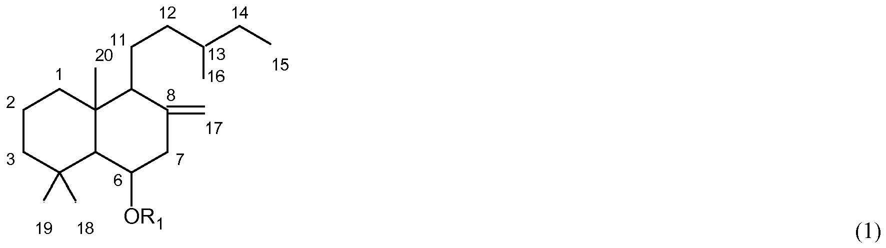 with R₁ being selected from hydrogen and C₁ to C₄ acyl, wherein optionally at least one double bond is present between the carbon atoms at positions 1 and 2, 2 and 3, 5 and 6, 6 and 7 and/or 14 and 15, wherein the carbon atoms at position 1, 2, 3, 7, 11, 12, 13, 14, 15, 16, 17, 18, 19 or 20 are connected to hydrogen atoms via single bonds or comprise at least one substitution, wherein optionally one carbon atom at position 18, 19 or 20 is replaced by COOH.

6. Bicyclic labdane diterpene for use or use according to claim 5, wherein substitutions at the carbon atoms at position 1, 2, 3, 7, 11, 12, 13, 14, 15, 16, 17, 18, 19 or 20 is selected from a halogen or-OR₂ with R₂ being hydrogen or C₁ to C₄ acyl and/or wherein at least one of positions 1, 2, 3 or 7 is a keto group.

7. Bicyclic labdane diterpene or use according to claim 5 or 6, wherein a double bond is present between carbon atoms at positions 14 and 15.

8. Bicyclic labdane diterpene or use according to one of claims 5 to 7, wherein the carbon atom at position 13 is substituted with -OR₂.

9. Bicyclic labdane diterpene or use according to one of claims 5 to 8, **characterized by** formula (2) 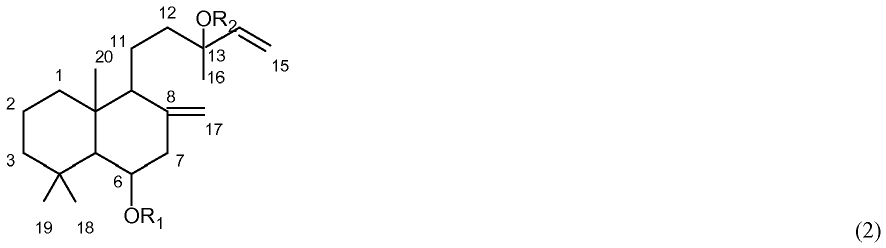 wherein R₁ and R₂ independent from each other are selected from hydrogen and C₁ to C₄ acyl, and wherein optional substitutions are present as defined in one of claims 7 or 8.

10. Bicyclic labdane diterpene or use according to one of claims 5 to 9, wherein the bicyclic labdane diterpene is larixol or larixol acetate.

## Patentansprüche

1. Bicyclisches Labdan-Diterpen zur Verwendung bei der Behandlung einer Lungenerkrankung oder einer Nierenerkrankung durch Blockieren des TRP(Transient Receptor Potential)-Kationenkanals 6.

2. Bicyclisches Labdan-Diterpen zur Verwendung nach Anspruch 1, wobei die Lungenerkrankung idiopathische oder sekundäre pulmonale Hypertonie, chronisch obstruktive Lungenerkrankung, eine allergische Atemwegserkrankung, pulmonale Vasokonstriktion oder ein akutes Atemnotsyndrom ist, oder die Nierenerkrankung eine fokalsegmentale Glomerulosklerose ist.

3. Verwendung des Labdan-Diterpens zum Blockieren des Calciumtransports über den TRP-Kationenkanal 6 ex vivo.

4. Verwendung nach Anspruch 3, wobei das bicyclische Labdan-Diterpen in einer wässrigen Lösung enthalten ist, die als Konservierungslösung zum Lagern und / oder zum Durchdringen von Gewebe eingesetzt wird.

5. Bicyclisches Labdan-Diterpen zur Verwendung nach Anspruch 1 oder 2 oder Verwendung nach Anspruch 3 oder 4, wobei das bicyclische Labdan-Diterpen eine Struktur nach der Formel (1) aufweist: 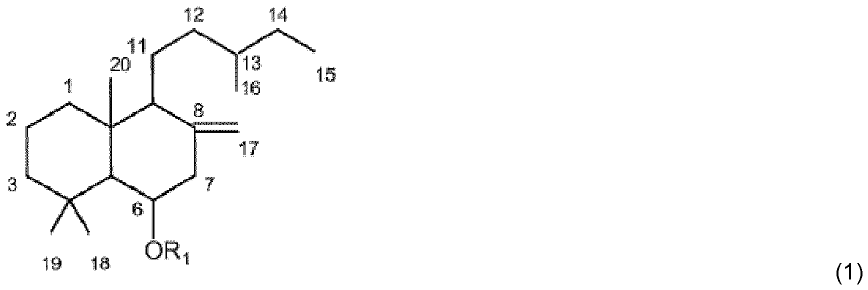 wobei R1 ausgewählt ist aus Wasserstoff und C₁- bis C₄-Acyl, wobei optional wenigstens eine Doppelbindung zwischen den Kohlenstoffatomen an den Positionen 1 und 2, 2 und 3, 5 und 6, 6 und 7 und / oder 14 und 15 vorliegt, wobei die Kohlenstoffatome an den Positionen 1, 2, 3, 7, 11, 12, 13, 14, 15, 16, 17, 18, 19 oder 20 über Einfachbindungen mit Wasserstoffatomen verbunden sind oder wenigstens eine Substitution aufweisen, wobei optional ein Kohlenstoffatom an Position 18, 19 oder 20 durch COOH substituiert ist.

6. Bicyclisches Labdan-Diterpen zur Verwendung nach Anspruch 5, wobei Substitutionen an den Kohlenstoffatomen an Position 1, 2, 3, 7, 11, 12, 13, 14, 15, 16, 17, 18, 19 oder 20 ausgewählt sind aus Halogen oder -OR₂, wobei R₂ Wasserstoff oder C₁- bis C₄-Acyl ist und / oder wobei wenigstens eine der Positionen 1, 2, 3 oder 7 eine Ketogruppe aufweist.

7. Bicyclisches Labdan-Diterpen oder Verwendung nach Anspruch 5 oder 6, wobei eine Doppelbindung zwischen Kohlenstoffatomen an Positionen 14 und 15 vorliegt.

8. Bicyclisches Labdan-Diterpen oder Verwendung nach einem der Ansprüche 5 bis 7, wobei das Kohlenstoffatom an Position 13 durch -OR₂ substituiert ist.

9. Bicyclisches Labdan-Diterpen oder Verwendung nach einem der Ansprüche 5 bis 8, **gekennzeichnet durch** die Formel (2) 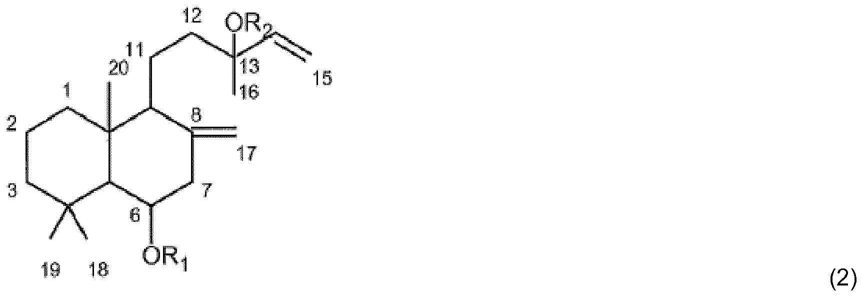 wobei R1 und R2 unabhängig voneinander ausgewählt sind aus der Gruppe bestehend aus Wasserstoff und C₁- bis C₄-Acyl und wobei optionale Substitutionen nach der Definition aus Anspruch 7 oder 8 vorliegen.

10. Bicyclisches Labdan-Diterpen oder Verwendung nach einem der Ansprüche 5 bis 9, wobei das bicyclische Labdan-Diterpen Larixol oder Larixolacetat ist.

## Revendications

1. Diterpène de labdane bicyclique pour une utilisation dans le traitement d'une maladie pulmonaire ou d'une maladie rénale par le blocage du transport du calcium par l'intermédiaire du canal cationique à potentiel récepteur transitoire 6.

2. Diterpène de labdane bicyclique pour une utilisation selon la revendication 1, où la maladie pulmonaire est l'hypertension pulmonaire idiopathique ou secondaire, la bronchopneumopathie chronique obstructive, une allergie respiratoire, la vasoconstriction pulmonaire ou un syndrome de détresse respiratoire aiguë ou la maladie rénale est une glomérulosclérose segmentaire focale.

3. Utilisation de diterpène de labdane bicyclique pour le blocage du transport du calcium par l'intermédiaire du canal cationique à potentiel récepteur transitoire 6 *ex vivo.*

4. Utilisation selon la revendication 3, où le diterpène de labdane bicyclique est compris dans une solution aqueuse qui est utilisée comme solution de conservation pour le stockage et/ou la perfusion de tissus.

5. Diterpène de labdane bicyclique pour une utilisation selon la revendication 1 ou 2, ou utilisation selon la revendication 3 ou 4, où le diterpène de labdane bicyclique présente une structure de formule (1) 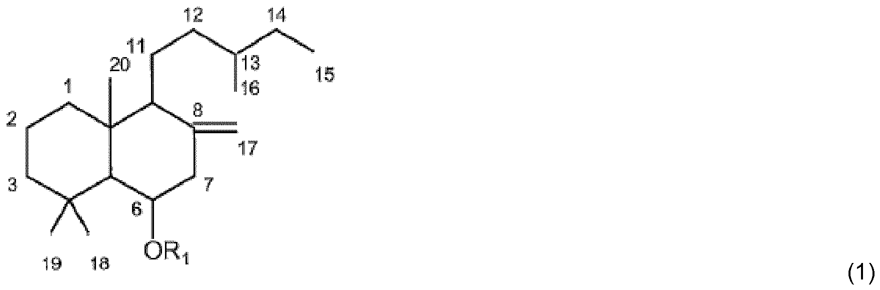 R₁ étant choisi parmi l'atome d'hydrogène et le groupe acyle en C₁ à C₄, où facultativement au moins une liaison double est présente entre les atomes de carbone en positions 1 et 2, 2 et 3, 5 et 6, 6 et 7 et/ou 14 et 15, où les atomes de carbone en position 1, 2, 3, 7, 11, 12, 13, 14, 15, 16, 17, 18, 19 ou 20 sont reliés à des atomes d'hydrogène par l'intermédiaire de liaisons simples ou comprennent au moins une substitution, où facultativement un atome de carbone en position 18, 19 ou 20 est remplacé par COOH.

6. Diterpène de labdane bicyclique pour une utilisation ou utilisation selon la revendication 5, où les substitutions au niveau des atomes de carbone en position 1, 2, 3, 7, 11, 12, 13, 14, 15, 16, 17, 18, 19 ou 20 sont choisies parmi un atome d'halogène ou -OR₂, R₂ étant un atome d'hydrogène ou un groupe acyle en C₁ à C₄ et/ou où au moins l'une des positions 1, 2, 3 ou 7 est un groupe céto.

7. Diterpène de labdane bicyclique ou utilisation selon la revendication 5 ou 6, où une liaison double est présente entre les atomes de carbone en positions 14 et 15.

8. Diterpène de labdane bicyclique ou utilisation selon l'une des revendications 5 à 7, où l'atome de carbone en position 13 est substitué par -OR₂.

9. Diterpène de labdane bicyclique ou utilisation selon l'une des revendications 5 à 8, **caractérisé par** la formule (2) 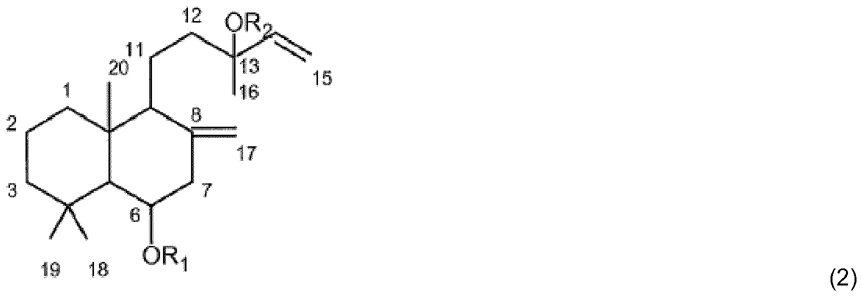 où R₁ et R₂ sont, indépendamment l'un de l'autre, choisis parmi un atome d'hydrogène et un groupe acyle en C₁ à C₄, et où des substitutions facultatives sont présentes conformément aux définitions données dans l'une des revendications 7 ou 8.

10. Diterpène de labdane bicyclique ou utilisation selon l'une des revendications 5 à 9, où le diterpène de labdane bicyclique est le larixol ou l'acétate de larixol.
